# FASCICULE DE BREVET EUROPEEN

(11) **EP 2 129 344 B1**
(45) Date de publication et mention de la délivrance du brevet: **21.10.2015**
(21) Numéro de dépôt: 08775712.6
(22) Date de dépôt: 11.03.2008
(51) Int. Cl.: A61F 5/448

(54) **RACCORD POUR APPAREILLAGE DE STOMIE, ET APPAREILLAGE DE STOMIE LE COMPORTANT**
STECKVERBINDER FÜR EINE OSTOMIEVORRICHTUNG UND OSTOMIEVORRICHTUNG DAMIT
CONNECTOR FOR AN OSTOMY APPARATUS, AND OSTOMY APPARATUS INCLUDING SAME

(30) Priorité: 15.03.2007 FR 0701874
(43) Date de publication de la demande: 09.12.2009
(73) Titulaire: B.Braun Medical SAS, 92100 Boulogne-Billancourt (FR)
(72) Inventeur: HOGARD, Aurélien, 64100 Bayonne (FR); LALET, Laurent, 64600 Anglet (FR)
(74) Mandataire: Ipside
(86) Numéro de dépôt international: PCT/FR2008/050411
(87) Numéro de publication internationale: WO 2008/129196

(56) Documents cités:
- EP-A- 0 630 626
- EP-A- 0 799 608
- EP-A- 1 108 404
- FR-A- 2 387 643
- US-A- 3 528 420

## Description

La présente invention concerne un raccord pour appareillage de stomie, et un appareillage de stomie qui le comporte.

Il existe de nombreux types de raccords d'appareillage de stomie, appartenant à deux principales catégories, les raccords mécaniques et les raccords collés. L'invention concerne un raccord de type mécanique.

Un raccord pour appareillage de stomie a deux fonctions principales, d'une part assurer le support du poids de l'appareillage, notamment lorsqu'elle contient des matières évacuées, et d'autre part empêcher le passage des liquides et des gaz (mauvaises odeurs) vers l'extérieur de l'appareillage. Une fonction supplémentaire, souvent présente à la demande des utilisateurs, est une fonction de sécurité assurée soit par un verrouillage de la fixation mécanique, soit par une fixation redondante de l'appareillage à son support afin qu'il ne puisse pas se séparer de ce support même en cas de défaillance du dispositif principal de fixation mécanique. Cette fonction est remplie par exemple lorsqu'un bruit audible par l'utilisateur est créé lorsque la fixation mécanique est enclenchée. Un raccord selon les caractéristiques du préambule de la revendication 1 est connu du document US-A-3 528 420.

Dans la plupart des raccords d'appareillage de stomie de type mécanique, les deux fonctions d'une part de support mécanique principal et d'autre part d'étanchéité sont remplies par des organes séparés. Plus précisément, un premier organe assure le maintien centré d'un premier organe de raccord sur un autre organe, et un joint d'étanchéité, tel qu'une lèvre déformable, assure l'étanchéité à un emplacement différent de celui du maintien mécanique. Les parties responsables de ce maintien mécanique ne sont pas élastiquement déformables, ou seulement de manière réduite pour permettre un enclenchement élastique, mais non pour assurer l'étanchéité.

Ces raccords de type mécanique, dans lesquels des parties rigides coopèrent pour le centrage et le maintien, ne présentent des propriétés élastiques qu'au niveau de la lèvre qui se déforme grâce à sa faible épaisseur, si bien qu'ils ne possèdent pas en général une souplesse suffisante pour le confort du patient.

On a déjà envisagé de réaliser des raccords d'appareillage de stomie ayant, sur un support fixé au patient, une gorge qui débouche radialement vers l'extérieur et dans laquelle vient s'accrocher une bague élastique. Etant donné l'orientation radiale de la gorge, la bague élastique doit présenter une certaine plage d'allongement élastique. Cependant, puisque cette bague est élastique, on conçoit que, lorsqu'une force est appliquée par le sac, la bague élastique a tendance à s'allonger et à se séparer du flasque qui délimite la gorge du support. Pour cette raison, comme décrit dans le document FR-A-2 387 643, la bague élastique n'est utilisée que pour une fonction d'étanchéité, s'opposant au passage des matières évacuées et des mauvaises odeurs, alors qu'un autre dispositif assure le support mécanique du poids du sac. Dans le document précité, ce dispositif de support mécanique est constitué d'une bague qui vient coincer un col formé par le sac contre la périphérie du flasque extérieur du support fixé sur l'utilisateur.

L'invention concerne un raccord d'appareillage de stomie dans lequel une bague élastique d'un organe vient se loger dans une gorge débouchant radialement d'un autre organe, et la bague assure à la fois l'étanchéité aux matières évacuées et aux mauvaises odeurs, et le support mécanique de l'appareillage, même lorsque celui-ci est remplie. Ce résultat est obtenu par disposition, autour de la bague élastique, d'un dispositif d'application du poids de l'appareillage à la seule partie de la bague élastique qui se trouve à la partie supérieure de la gorge ; cette partie supérieure de bague travaille ainsi en compression et assure ses fonctions d'étanchéité et de support mécanique d'autant plus efficacement que le poids de l'appareillage augmente. Ce dispositif est par exemple une zone déformable qui décharge la partie inférieure de la bague du poids de l'appareillage, si bien que cette partie inférieure n'a pas tendance à s'écarter du fond de la gorge.

Plus précisément, l'invention concerne un raccord pour appareillage de stomie, du type qui comprend un premier organe solidaire d'un support destiné à être fixé à la peau d'un utilisateur et un second organe solidaire d'une poche formant réceptacle ; selon l'invention, le premier organe délimite une ouverture, il est formé d'un matériau souple afin qu'il puisse s'adapter aux variations de forme de la partie du corps de l'utilisateur à laquelle est fixé le support, et il délimite une gorge continue débouchant radialement autour de l'ouverture, et le second organe comporte une bague élastique entourant une ouverture et une joue disposée globalement dans un plan entre la bague élastique et une périphérie distante de la bague, le diamètre interne de la bague élastique du second organe à un état de repos étant inférieur au diamètre du fond de la gorge du premier organe, et la joue étant destinée à être solidarisée à une feuille d'une poche. Le second organe comporte en outre, entre la bague et la joue, un dispositif d'application du poids de l'appareillage à la seule partie de la bague élastique qui se trouve à la partie supérieure de la gorge.

De préférence, le dispositif d'application du poids de l'appareillage à la seule partie de la bague élastique qui se trouve à la partie supérieure de la gorge est une zone déformable de découplage telle qu'une partie de joue adjacente à la bague élastique et une partie de joue adjacente à la périphérie présentent un déplacement relatif dans ledit plan lorsque la joue est soumise à une force agissant dans son plan.

Dans un mode de réalisation, la zone déformable est formée tout autour de la bague dans chaque direction radiale, et la zone déformable a une capacité d'allongement plus grande que sa capacité de rétrécissement. Dans ce cas, la zone déformable a de préférence sa plus grande capacité de déformation du côté destiné à se trouver vers le bas sur un utilisateur debout.

Dans un autre mode de réalisation, la zone déformable entoure une partie seulement de la bague du côté destiné à se trouver vers le bas sur un utilisateur debout.

De préférence, le second organe possède au moins une patte de traction destinée à appliquer à la bague élastique une force dirigée radialement vers l'extérieur.

De préférence, la patte au moins est fixée à un emplacement qui est plus vers l'intérieur de la joue que la zone déformable. Par exemple, la patte de traction est fixée à la bague élastique.

De préférence, le second organe comporte trois pattes de traction espacées d'environ 13° et disposées du côté opposé au côté destiné à se trouver vers le bas sur un utilisateur debout.

De préférence, le second organe formé par la bague élastique, le dispositif d'application du poids de l'appareillage, la joue et les pattes le cas échéant est formé en une seule pièce. Par exemple, le second organe est formé en une seule pièce par moulage par injection.

De préférence, la gorge du premier organe est délimitée, du côté destiné à être le plus éloigné du support, par un flasque extérieur dont la dimension radiale vers l'extérieur de l'ouverture est accrue du côté destiné à se trouver vers le bas sur un utilisateur debout.

De préférence, la dimension radiale du flasque extérieur du premier organe est accrue à chaque emplacement correspondant à une patte du second organe.

De préférence, le premier organe est formé d'un élastomère.

De préférence, le raccord comporte en outre un organe redondant de fixation mécanique du premier organe au second organe. Par exemple, l'organe redondant est choisi parmi un organe de fixation à boucles et crochets et un organe à bouton-pression.

L'invention concerne aussi un appareillage de stomie qui comprend au moins deux épaisseurs de feuille délimitant un réceptacle, et telle qu'une feuille est fixée à un organe constitué par un second organe d'un raccord selon l'un des paragraphes précédents.

De préférence, la fixation du second organe à la feuille est choisie parmi un soudage et un collage.

Un premier avantage du raccord selon l'invention est qu'il peut être peu coûteux parce que chacun des deux organes de raccord peut être facilement formé en une seule pièce par moulage par injection.

Un second avantage du raccord selon l'invention est que les deux organes du raccord peuvent être souples, et donnent donc un bon confort à l'utilisateur.

Un troisième avantage du raccord selon l'invention est que les parties élargies de flasque peuvent donner à l'utilisateur une sensation suffisante de sécurité pour qu'il soit possible de supprimer tout dispositif redondant de fixation.

D'autres caractéristiques et avantages de l'invention seront mieux compris à la lecture de la description qui va suivre, faite en référence aux dessins annexés sur lesquels :
la figure 1 est une vue en plan d'un premier organe de raccord d'appareillage de stomie selon l'invention, destiné à être fixé de façon permanente à un support collé à la peau d'un utilisateur ;
la figure 2 est une coupe suivant la ligne A_A de la figure 1 ;
la figure 3 est une vue en plan d'un second organe de raccord d'appareillage de stomie selon l'invention, destiné à coopérer avec le premier organe des figures 1 et 2 ;
la figure 4 est une coupe suivant la ligne B_B de la figure 3 ;
la figure 5 est un agrandissement de la partie gauche de la figure 4 ;
la figure 6 est une coupe d'un raccord d'appareillage de stomie selon l'invention en position de coopération, suivant les lignes de coupe A_A et B_B des figures 1 et 3 ;
la figure 7 est analogue à la figure 1 mais représente une variante de premier organe de raccord d'appareillage de stomie selon l'invention ; et
la figure 8 est analogue à la figure 3 mais représente une variante de second organe de raccord d'appareillage de stomie selon l'invention.

Le raccord pour appareillage de stomie selon l'invention est destiné à être utilisé dans les conditions habituelles d'utilisation de ces appareillages. La poche de ces appareillages a en général un volume maximal inférieur à un litre. Le poids de l'appareillage rempli est donc toujours inférieur à 1 kg. La force appliquée pour vérifier l'étanchéité et l'herméticité de l'appareillage doit donc être au moins deux fois supérieure à celle qui correspond à ce poids, c'est-à-dire au moins égale à 20 N. On utilise aussi couramment un test statique d'étanchéité dans lequel une force de 200 N est appliquée à un appareillage rempli d'eau, disposé horizontalement.

Avant de décrire le raccord pour appareillage de stomie selon l'invention en position d'utilisation, on considère séparément ses deux organes.

Le premier organe 10 du raccord comprend une joue 12 destinée à être maintenue de manière permanente par une rondelle de gomme collée à la peau d'un utilisateur. Cette joue 12 entoure une ouverture centrale 13 représentée sous forme circulaire.

Un flasque 14, distant de la joue 12, délimite avec celle-ci une gorge 16 tournée radialement vers l'extérieur. De préférence, le flasque 14 a une largeur accrue à divers emplacements de sa périphérie, notamment dans la partie qui est destinée à se trouver vers le bas lorsque l'utilisateur est debout, comme indiqué par la référence 18, et à deux emplacements 20, séparés de 140°environ, de la partie du flasque 14 destinée à se trouver vers le haut lorsque l'utilisateur est debout.

Le premier organe 10 a une bonne souplesse lui permettant de s'adapter à la courbure de la peau de l'utilisateur. Il peut ainsi être formé d'un élastomère de copolymère d'acétate de vinyle et d'une oléfine, telle que l'éthylène et le propylène.

Le second organe 22 comprend essentiellement, autour d'une ouverture centrale 23, une bague élastique 24 solidaire d'une joue 26. La bague élastique 24 a de préférence une section circulaire, et le diamètre interne de l'ouverture 23 délimitée par la bague élastique à un état de repos est inférieur au diamètre du fond de la gorge 16 du premier organe.

Le second organe 22 comprend également, entre la joue 26 et la bague élastique 24, une zone déformable 28 représentée sous forme d'un pli en U formé d'un côté de la joue 16. Plus précisément, la zone déformable 28 s'étend radialement autour de la bague élastique 24, de manière à relier la périphérie externe de ladite bague 24, dirigée vers l'extérieur par rapport à l'ouverture 23, à la périphérie interne de la joue 26, dirigée vers l'ouverture 23.

Cette zone déformable constitue le dispositif d'application du poids de l'appareillage à la seule partie de la bague élastique qui se trouve à la partie supérieure de la gorge selon l'invention, dans le mode de réalisation considéré. Cependant, ce dispositif peut avoir diverses autres formes. Par exemple, il peut être formé d'une zone déformable ayant deux plis consécutifs, adjacents ou en Z, c'est-à-dire tournés l'un vers l'intérieur et l'autre vers l'extérieur.

Dans une variante, cette zone déformable peut aussi ne s'étendre que sur une partie de la périphérie externe de la bague élastique 24, correspondant à la partie inférieure sur l'utilisateur debout. Dans ce cas, dans la partie de la périphérie externe de la bague élastique 24 sans zone déformable, ladite bague 24 est directement solidaire de la périphérie interne de la joue 26.

En outre, le second organe comprend de préférence trois pattes 30' 30" 30"' qui dépassent radialement vers l'extérieur de la bague élastique 24 dont elles sont solidaires (elles peuvent aussi être solidaires de la partie de joue placée entre la bague élastique 24 et la zone déformable 28).

Bien que les pattes 30' 30" 30'" soient représentées comme dépassant de la périphérie de la joue 26, elles peuvent aussi ne pas dépasser celle-ci.

Le rôle des pattes 30' 30" 30'" est de permettre à l'utilisateur de les saisir pour étirer la bague élastique 24 lors de sa mise en place dans la gorge du premier organe 10 ou de son extraction de cette gorge, comme décrit plus en détail dans la suite.

Le second organe 22 peut être formé par injection d'une matière plastique, par exemple d'un copolymère d'acétate de vinyle et d'éthylène, ou de préférence d'un copolymère de type styrène-éthylène-butylène-styrène ou styrène-isoprène-styrène.

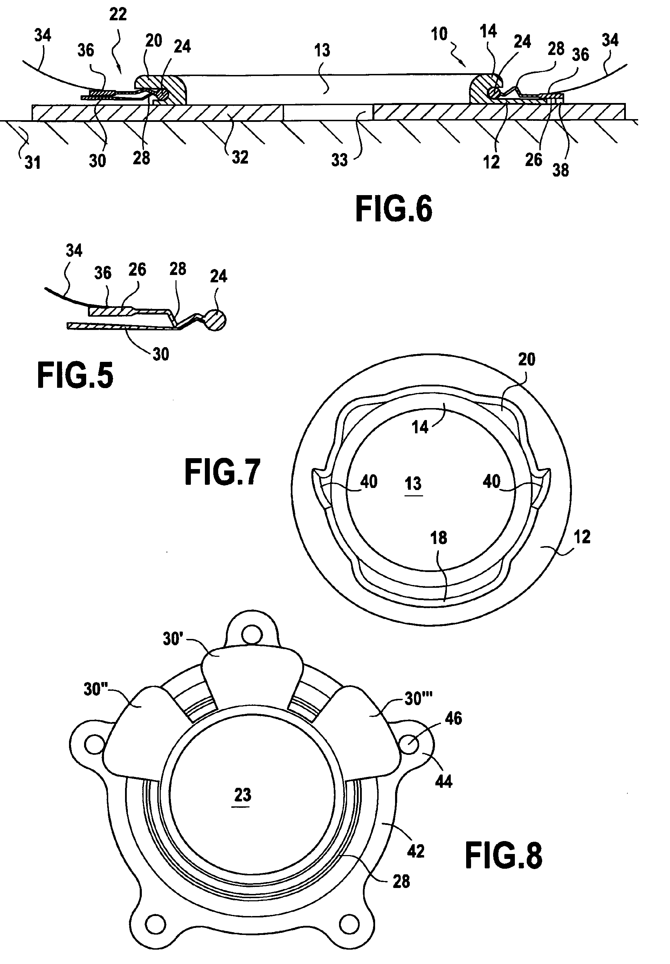

Avant de décrire l'utilisation du raccord, on considère, en référence à la figure 6, la coopération des deux organes.

Sur la figure 6, un support formant étrier est constitué d'une rondelle de gomme 32 collée à la peau 31 de l'utilisateur, et ayant une ouverture centrale 33 dans laquelle se trouve la stomie. Le premier organe 10 du raccord a sa joue 12 fixée de manière permanente, par collage dans ce cas, à la rondelle de gomme 32. Le support est donc entièrement constitué par la rondelle de gomme 32 et le premier organe 14 du raccord. L'ouverture 13 du premier organe du raccord correspond à l'ouverture 33 de la rondelle de gomme.

La partie périphérique de la joue 26 du second organe 22 est soudée de manière étanche en 36 au bord d'une ouverture d'une feuille 34 de la poche, et sa bague élastique 24 est logée dans la gorge du premier organe de raccord.

Dans l'hypothèse où l'utilisateur souhaite un dispositif redondant de fixation, on a aussi indiqué sous forme schématique un dispositif éventuel de fixation 38, du type à boucles et crochets, fixé entre la joue 12 du premier organe, qui peut alors être localement étendue comme cela est représenté à droite sur la figure 6, et la partie extérieure de la joue 26 du second organe. Ce dispositif 38 peut aussi représenter des boutons-pressions ou tout autre dispositif convenable. La figure 8, décrite dans la suite, représente une variante de dispositif redondant de fixation. Cependant, il n'est aucunement nécessaire selon l'invention, sinon pour donner à l'utilisateur une impression de double sécurité.

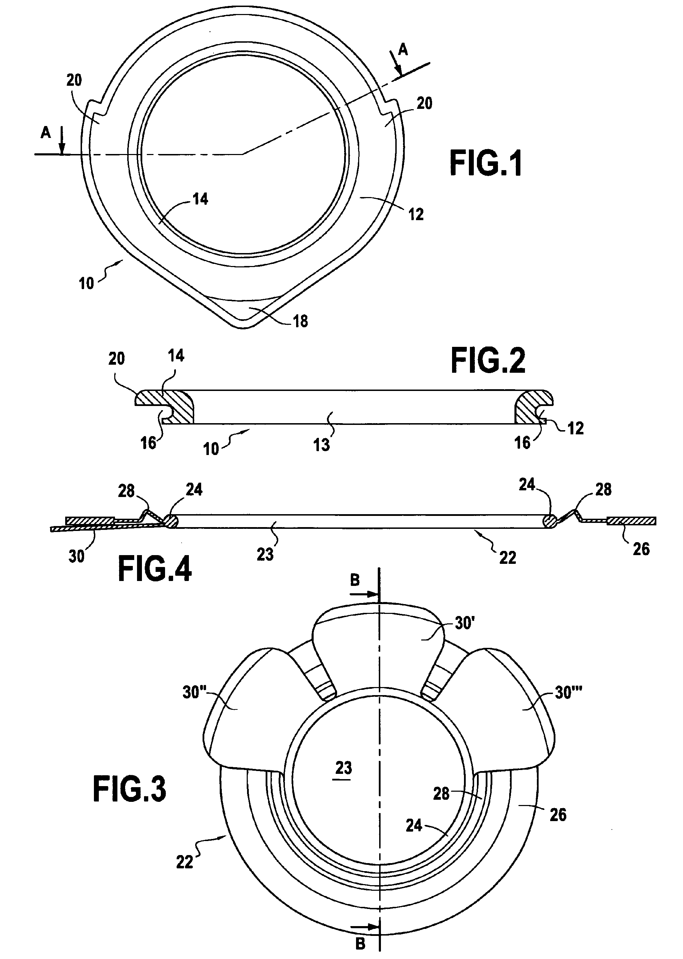

Lors de la mise en place d'un appareillage, le support formé de la rondelle de gomme 32 et du premier organe 10 est déjà collé à la peau l'utilisateur avec la disposition indiquée sur la figure 1, c'est-à-dire que l'élargissement 18 du flasque 14 est dirigé vers le bas.

L'utilisateur saisit l'appareillage par les deux pattes 30" 30"' du second organe 22 du raccord et place la partie inférieure de la bague élastique 24 sous la partie élargie 18. Par une traction vers le haut, il vient plaquer cette partie inférieure de la bague élastique contre le fond de la gorge à la partie inférieure. Ensuite, il tire les pattes 30" 30"' vers le haut pour étirer la bague élastique 24 et la faire passer au-dessus des deux élargissements 20 du flasque 14. Enfin, l'utilisateur saisit la patte supérieure 30' et la tire vers le haut pour amener la partie correspondante de la bague élastique 24 dans la gorge 16 en relâchant la patte 30'. La bague élastique 24, dont le diamètre interne est inférieur à celui du fond de la gorge 16, vient se plaquer élastiquement contre la gorge tout autour de celle-ci. L'étanchéité est ainsi assurée.

Lorsque la poche se remplit progressivement, le poids appliqué à la joue 26 augmente. La partie extérieure de la joue 26 a donc tendance à descendre de plus en plus. A ce moment, la zone déformable 28 se comprime à la partie supérieure et s'écarte à la partie inférieure. Etant donné la forme en V, en U ou en Z de la zone déformable 28, la partie supérieure de la joue 26 se comprime et supporte le poids de l'appareillage avant que la partie inférieure ne soit suffisamment allongée pour transmettre une force conséquente à la partie inférieure de la bague élastique 24.

En conséquence, le poids est transmis par la joue 26 à la partie supérieure de la bague élastique 24, sous forme d'une force de compression de la bague élastique contre le fond de la gorge, sans qu'aucune force pratiquement n'ait tendance à écarter la partie inférieure de la bague élastique 24 de la gorge.

Lorsque la poche doit être retirée du support, l'utilisateur saisit d'abord la patte supérieure 30', la tire vers le haut pour faire passer la partie correspondante de bague élastique 24 en dehors de la gorge 16. Puis il saisit les pattes latérales 30" et 30"' pour désengager progressivement le reste de la bague élastique 24, par dessus les élargissements 20, de la gorge 16, puis abaisse la poche pour la retirer et la vider.

Bien qu'on ait muni la joue 26 d'une région déformable 28 s'étendant sur toute sa périphérie externe, cette zone déformable peut n'être présente qu'à certains emplacements, par exemple à la moitié inférieure de la périphérie externe de la joue 26. Par ailleurs, d'autres zones déformables peuvent être envisagées. Par exemple, l'épaisseur de la joue 26 peut être augmentée à la partie supérieure ou suivant un trajet préférentiel de transmission du poids de l'appareillage. Cependant, une telle solution n'est pas forcément confortable pour l'utilisateur, car la joue 26 peut devenir trop rigide localement.

La figure 7, qui est analogue à la figure 1, représente une variante de premier organe de raccord d'appareillage de stomie selon l'invention, les références identiques à celles de la figure 1 désignant des éléments analogues.

Dans cette variante, le flasque 14 de l'appareillage des figures 1 à 6 comporte non seulement des élargissements 18, 20, mais aussi des élargissements 40 en forme de pointe orientée vers le haut.

Lorsque l'utilisateur met en place l'appareillage en tirant la bague élastique par les pattes, il peut effectuer une pause à mi-course lorsque la bague élastique a dépassé les élargissements 40, mais n'a pas encore atteint les élargissements 20. Il peut par exemple tirer une patte 30" '30"' d'un seul côté jusqu'à faire passer la bague sur l'élargissement 40 de ce côté, puis éventuellement lâcher ladite patte 30" 30"' de ce côté et saisir une patte 30" 30"' du côté opposé pour la faire passer sur l'autre élargissement.

Il est ainsi possible de mettre en place ou de retirer l'appareillage en n'utilisant qu'une seule main. Bien qu'on ait représenté un seul élargissement 40 de chaque côté, il est possible d'en incorporer plusieurs qui constituent alors une sorte de crémaillère.

La figure 8, qui est analogue à la figure 3, représente une variante de second organe de raccord d'appareillage de stomie selon l'invention, les références identiques à celles de la figure 3 désignant des éléments analogues.

Sur la figure 8, un second organe de raccord comporte une joue 42 qui comporte des oreilles 44 à sa périphérie. Ces oreilles 44 ont chacune un trou 46 destiné au passage d'un plot (non représenté) d'un premier organe analogue à celui de la figure 1 dont la joue 12 porte de tels plots. Lorsque l'utilisateur a mis en place la bague élastique 24 dans la gorge 16, il peut introduire un ou plusieurs plots dans un ou plusieurs trous de la joue 42. L'ouverture du raccord est alors empêchée tant que les plots n'ont pas été retirés des trous correspondants.

Bien qu'on ait représenté un raccord dont la bague élastique 24 porte trois pattes de traction 30' 30" 30"', il est possible de n'utiliser qu'une seule patte dirigée vers le haut, ou deux pattes séparées de 90°. De même, il est possible de disposer un nombre de pattes supérieur à trois, de manière que l'utilisateur puisse choisir les pattes qu'il considère comme les plus commodes pour la mise en place ou l'extraction.

## Revendications

1. Raccord pour appareillage de stomie, comprenant un premier organe (10) solidaire d'un support destiné à être fixé à la peau d'un utilisateur et un second organe (22) solidaire d'une poche formant réceptacle,
- le premier organe (10) délimite une ouverture (13), il est formé d'un matériau souple afin qu'il puisse s'adapter aux variations de forme de la partie du corps de l'utilisateur à laquelle est fixé le support, et il délimite une gorge continue (16) débouchant radialement autour de l'ouverture (13), et
- le second organe (22) comporte une bague élastique (24) entourant une ouverture (23) et une joue (26) disposée globalement dans un plan entre la bague élastique (24) et une périphérie distante de la bague (24), le diamètre interne de la bague élastique (24) du second organe (22) à un état de repos étant inférieur au diamètre du fond de la gorge (16) du premier organe (10), et la joue (26) étant destinée à être solidarisée à une feuille d'une poche,
**caractérisé en ce que** le second organe comporte également un dispositif d'application du poids de l'appareillage à la seule partie de la bague élastique (24) qui se trouve à la partie supérieure de la gorge (16), ledit dispositif d'application du poids de l'appareillage étant formé d'une zone déformable (28) s'étendant entre la bague et la joue et sur un périmètre au moins partiel de la bague, ladite zone déformable présentant à l'état de repos une pliure en V, en U ou en Z et ladite zone déformable étant apte à être comprimée du côté supérieur et étirée du côté inférieur de la bague lorsque le poids de la poche augmente.

2. Raccord selon la revendication 1, **caractérisé en ce que** la zone déformable (28) est telle qu'une partie de joue (26) adjacente à la bague élastique (24) et une partie de joue (26) adjacente à la périphérie présentent un déplacement relatif dans ledit plan lorsque la joue (26) est soumise à une force agissant dans son plan.

3. Raccord selon l'une des revendications 1 à 2, **caractérisé en ce que** la zone déformable (28) a sa plus grande capacité de déformation du côté destiné à se trouver vers le bas sur un utilisateur debout.

4. Raccord selon l'une quelconque des revendications 1 à 3, **caractérisé en ce que** le second organe (22) possède au moins une patte (30' 30" 30"') de traction destinée à appliquer à la bague élastique (24) une force dirigée radialement vers l'extérieur.

5. Raccord selon la revendication 4, **caractérisé en ce que** la patte est fixée à un emplacement qui est plus vers l'intérieur de la joue (26) que la zone déformable (28).

6. Raccord selon l'une des revendications 4 et 5, **caractérisé en ce que** le second organe (22) comporte trois pattes (30' 30" 30"') de traction espacées d'environ 13° et disposées du côté opposé au côté destiné à se trouver vers le bas sur un utilisateur debout.

7. Raccord selon l'une quelconque des revendications 1 à 6, **caractérisé en ce que** le second organe (22) formé par la bague élastique (24), la joue (26), la zone déformable et les pattes (30' 30" 30"') le cas échéant est formé en une seule pièce.

8. Raccord selon l'une quelconque des revendications 1 à 7, **caractérisé en ce que** la gorge (16) du premier organe (10) est délimitée, du côté destiné à être le plus éloigné du support, par un flasque extérieur (14) dont la dimension radiale vers l'extérieur de l'ouverture (13) est accrue du côté destiné à se trouver vers le bas sur un utilisateur debout.

9. Raccord selon la revendication 8, **caractérisé en ce que** la dimension radiale du flasque extérieur (14) du premier organe est accrue à chaque emplacement correspondant à une patte (30' 30" 30"') du second organe (22).

10. Appareillage de stomie, comportant au moins deux épaisseurs de feuille délimitant un réceptacle, **caractérisé en ce que** l'appareillage comporte un raccord selon l'une quelconque des revendications précédentes et **en ce qu'**une feuille est fixée ou dit second organe (22).

## Patentansprüche

1. Steckverbinder für eine Ostomievorrichtung, der ein erstes Element (10), das mit einem Träger fest verbunden ist, der zur Befestigung an der Haut eines Benutzers ausgeführt ist, und ein zweites Element (22), das mit einer einen Behälter bildenden Tasche fest verbunden ist, umfasst,
- wobei das erste Element (10) eine Öffnung (13) begrenzt, es aus einem weichen Material hergestellt ist, so dass es sich an die Variationen der Form des Körperteils des Benutzers, an dem der Träger befestigt ist, anpassen kann, und es eine durchgängige Nut (16) begrenzt, die radial um die Öffnung (13) geöffnet ist, und
- wobei das zweite Element (22) einen elastischen Ring (24), der eine Öffnung (23) umgibt, und eine Wange (26), die allgemein in einer Ebene zwischen dem elastischen Ring (24) und einem vom Ring (24) entfernten Rand angeordnet ist, aufweist, der Innendurchmesser des elastischen Rings (24) des zweiten Elements (22) in einem Ruhezustand unter dem Durchmesser des Bodens der Nut (16) des ersten Elements (10) liegt und die Wange (26) zur festen Verbindung mit einer Schicht einer Tasche ausgeführt ist,
**dadurch gekennzeichnet, dass** das zweite Element des Weiteren ein Mittel zum Anbringen des Gewichts des Geräts nur an dem Teil des elastischen Rings (24), der sich im oberen Teil der Nut (16) befindet, umfasst, wobei das Mittel zum Anbringen des Gewichts des Geräts in einem verformbaren Bereich (28) ausgebildet ist, der sich zwischen dem Ring und der Wange und mindestens auf einem Teilumfang des Rings erstreckt, wobei der verformbare Bereich im Ruhezustand eine V-, U- oder Z-Falzung aufweist und der verformbare Bereich auf der Oberseite zusammengedrückt und auf der Unterseite des Rings auseinandergezogen werden kann, wenn sich das Gewicht der Tasche erhöht.

2. Steckverbinder nach Anspruch 1, **dadurch gekennzeichnet, dass** der verformbare Bereich (28) derart ist, dass ein Teil der Wange (26) neben dem elastischen Ring (24) und ein Teil der Wange (26) neben dem Rand bezüglich einander in der Ebene verschoben werden, wenn die Wange (26) einer in ihrer Ebene wirkenden Kraft unterworfen wird.

3. Steckverbinder nach einem der Ansprüche 1-2, **dadurch gekennzeichnet, dass** der verformbare Bereich (28) seine größte Verformungskapazität auf der Seite hat, die sich bei einem stehenden Benutzer unten befinden soll.

4. Steckverbinder nach einem der Ansprüche 1-3, **dadurch gekennzeichnet, dass** zweite Element (22) mindestens eine Zuglasche (30' 30" 30"') zum Aufbringen einer radial nach außen gerichteten Kraft auf den elastischen Ring (24) besitzt.

5. Steckverbinder nach Anspruch 4, **dadurch gekennzeichnet, dass** die Lasche an einer Stelle befestigt ist, die sich näher dem Inneren der Wange (26) befindet als der verformbare Bereich (28).

6. Steckverbinder nach einem der Ansprüche 4 und 5, **dadurch gekennzeichnet, dass** das zweite Element (22) drei Zuglaschen (30' 30" 30"") aufweist, die um etwa 13° beabstandet und auf der Seite angeordnet sind, die der Seite, die sich bei einem stehenden Benutzer unten befinden soll, gegenüberliegt.

7. Steckverbinder nach einem der Ansprüche 1-6, **dadurch gekennzeichnet, dass** das durch den elastischen Ring (24), die Wange (26), den verformbaren Bereich und die Laschen (30' 30" 30"") gebildete zweite Element (22) gegebenenfalls einstückig ausgebildet ist.

8. Steckverbinder nach einem der Ansprüche 1-7, **dadurch gekennzeichnet, dass** die Nut (16) des ersten Elements (10) auf der Seite, die so ausgeführt ist, dass sie sich am weitesten vom Träger entfernt befindet, durch einen äußeren Flansch (14) begrenzt wird, dessen radiale Abmessung zum Äußeren der Öffnung (13) auf der Seite, die sich bei einem stehenden Benutzer unten befinden soll, zunimmt.

9. Steckverbinder nach Anspruch 8, **dadurch gekennzeichnet, dass** die radiale Abmessung des äußeren Flanschs (14) des ersten Elements an jeder Stelle, die einer Lasche (30' 30" 30"') des zweiten Elements (22) entspricht, zunimmt.

10. Ostomievorrichtung, die mindestens zwei Lagen einer einen Behälter begrenzenden Schicht umfasst, **dadurch gekennzeichnet, dass** die Vorrichtung einen Steckverbinder nach einem der vorhergehenden Ansprüche umfasst und dass eine Schicht am zweiten Element (22) befestigt ist.

## Claims

1. Connector for an ostomy device, comprising a first element (10) that is continuous with a support designed to be attached to the skin of a user and a second element (22) that is continuous with a bag forming a receptacle,
- the first element (10) surrounds an opening (13), it is made of a flexible material so that it can conform to variations in the shape of the part of the user's body to which the support is attached, and it surrounds a continuous groove (16) that opens radially around the opening (13), and
- the second element (22) comprises an elastic ring (24) surrounding an opening (23) and a plate (26) arranged throughout in a plane between the elastic ring (24) and a distant outer edge of the ring (24), with the inner diameter of the elastic ring (24) of the second element (22) at rest being less than the diameter of the bottom of the groove (16) of the first element (10), with the plate (26) being designed to be joined integrally to a sheet of a bag, **characterized in that** the second element also comprises a means for applying the weight of the device only to the part of the elastic ring (24) that is located on the upper part of the groove (16), said means for applying the weight of the device being formed of an area whose shape can be modified (28) extending between the ring and the plate and over an at least partial perimeter of the ring, said area whose shape can be modified having, in the state of rest, a V-shaped, U-shaped or Z-shaped fold, and said area whose shape can be modified being able to be compressed at the top and stretched at the bottom of the ring as the weight of the bag increases.

2. Connector according to Claim 1, **characterized in that** the area whose shape can be modified (28) is such that a part of the plate (26) adjacent to the elastic ring (24) and a part of the plate (26) adjacent to the outer edge are displaced relative to one another in said plane when the plate (26) is subjected to a force acting in its plane.

3. Connector according to either of Claims 1 and 2, **characterized in that** the area whose shape can be modified (28) has its greatest capacity for modification in shape on the side designed to face downward when the user is standing.

4. Connector according to any one of Claims 1 to 3, **characterized in that** the second element (22) has at least one traction tab (30' 30" 30) designed to apply to the elastic ring (24) a force directed radially outward.

5. Connector according to Claim 4, **characterized in that** the tab is attached to a site that is closer to the inside of the plate (26) than the area whose shape can be modified (28).

6. Connector according to either of Claims 4 and 5, **characterized in that** the second element (22) comprises three traction tabs (30' 30" 30) separated by approximately 13° and arranged on the side opposite the side designed to face downward when the user is standing.

7. Connector according to any one of Claims 1 to 6, **characterized in that** the second element (22) formed by the elastic ring (24), the plate (26), the area whose shape can be modified and the tabs (30' 30" 30), if applicable, is formed in a single piece.

8. Connector according to any one of Claims 1 to 7, **characterized in that** the groove (16) of the first element (10) is surrounded, on the side designed to be farthest from the support, by an outer flange (14) whose radial dimension towards the outside of the opening (13) is increased on the side designed to face downward when the user is standing.

9. Connector according to Claim 8, **characterized in that** the radial dimension of the outer flange (14) of the first element is increased at each site corresponding to a tab (30' 30" 30) of the second element (22).

10. Ostomy device, comprising at least two thicknesses of sheet surrounding a receptacle, **characterized in that** the device comprises a connector according to any one of the preceding claims, and **in that** one sheet is attached to said second element (22).
